(19) Europäisches Patentamt
European Patent Office
Office européen des brevets

(11) Veröffentlichungsnummer: **0 387 755 A1**

(12) **EUROPÄISCHE PATENTANMELDUNG**

(21) Anmeldenummer: 90104639.1

(22) Anmeldetag: 12.03.90

(51) Int. Cl.⁵: **A61B 17/22**

(30) Priorität: 17.03.89 DE 8903333 U

(43) Veröffentlichungstag der Anmeldung:
**19.09.90 Patentblatt 90/38**

(84) Benannte Vertragsstaaten:
**AT CH DE FR GB IT LI NL SE**

(71) Anmelder: **Schott Glaswerke**
**Hattenbergstrasse 10**
**D-6500 Mainz(DE)**
(84) **CH DE FR IT LI NL SE AT**

Anmelder: **CARL-ZEISS-STIFTUNG**
**Schott Glaswerke Hattenbergstrasse 10**
**D-6500 Mainz 1(DE)**
(84) **GB**

(72) Erfinder: **Müller, Gerhard, Prof.-Dr.**
**An der Rehwiese 8**
**D-1000 Berlin 38(DE)**
Erfinder: **Kar, Hasan, Dipl.-Phys.**
**Schildhornstrasse 22**
**D-1000 Berlin 41(DE)**
Erfinder: **Dörschel, Klaus, Dr.**
**Himbeersteig 16**
**D-1000 Berlin 38(DE)**
Erfinder: **Schönborn, Karl-Heinz, Dr.**
**Konrad-Adenauer-Strasse 27**
**D-6500 Mainz 41(DE)**

(74) Vertreter: **Dr. Fuchs, Dr. Luderschmidt**
**Dipl.-Phys. Seids, Dr. Mehler Patentanwälte**
**Abraham-Lincoln-Strasse 7**
**D-6200 Wiesbaden(DE)**

(54) **Kathetersystem für die Gefässrekanalisation im menschlichen Körper.**

(57) In einem Laserkathetersystem zur Rekanilisation von durch Ablagerungen praktisch vollständig verschlossener Gefäßabschnitte im menschlichen Körper ist ein in den Innenkanal (14) des Hauptkatheters einführbarer und über das distale Ende hinaus verschiebbarer Pilotkatheter (10) vorgesehen, der einen Pilotlichtleiter (28) enthält, in dessen promximales Ende gepulstes Laserlicht einkoppelbar ist. Mittels einer ersten Strahlweicheneinrichtung in der Einkoppelanordnung läßt sich ein Anteil der Laserstrahlung aus einer Laserlichtquelle für die Einkopplung in den Pilotkatheter abzweigen. Die in die Lichtleiter des Haupkatheters reflektierte Strahlung wird in der Strahlweicheneinrichtung von der in den Hauptkatheter eintretenden Laserstrahlung getrennt und kann einer Auswerteinheit zugeführt werden. Über eine zweite Strahlweicheneinrichtung wird die in den Pilotkatheter reflektierte Strahlung einer Auswerteinheit zugeführt. Bei eingeführtem Pilotkatheter kann sowohl durch den Innenkanal des Hauptkatheters (14) als auch durch den Innenraum des Pilotkatheters (26) Spülfluid gepumpt werden.

Fig. 3

# KATHETERSYSTEM FÜR DIE GEFÄSSREKANALISATION IM MENSCHLICHEN KÖRPER

Die Erfindung betrifft ein Kathetersystem, insbesondere für die Gefäßrekanilisation im menschlichen Körper mit einer ringförmigen Lichtleiteranordnung zum Übertragen von Laserlicht nach dem Oberbegriff des Patentanspruches 1.

Zur Beseitigung der Folgen von durch Ablagerungen obstruierte Blutgefäße ist neben die herkömmlichen Methoden der chirurgischen Behandlung durch Austausch von Gefäßabschnitten bzw. Anordnung von Bypasskanälen und die Aufweitung verengter Gefäßabschnitte durch Ballondilatationskatheter die Rekanalisierung der Gefäße durch Ablation der Ablagerungen mittels Laserstrahlung getreten. Hierbei werden in das Gefäßsystem einführbare Katheter verwendet, welche Lichtleiter zum Transport der Laserenergie enthalten.

Es sind ferner Hilfsmethoden entwickelt worden, mit deren Hilfe das Erkennen der richtigen Behandlungsposition des distalen Endes der Laserlichtleiter im Gefäßsystem möglich ist, und Methoden, mit denen die Behandlungsstelle visuell mittels Lichtleiter-Bildübertragung durch den Katheter beobachtet werden kann.

Grundsätzliche Probleme und deren mögliche Lösungen bei der Rekanilisation von Gefäßen mittels Laserenergie sind in der EP-OS 0195375 angesprochen. Dieser Druckschrift sind Vorschläge zu entnehmen, wie durch optische Hilfsmittel am distalen Ende des Laserkatheters die Laserstrahlung mit gewünschter Energiedichte und möglichst in für die Gefäßwand unschädlicher weise auf die zu beseitigenden Ablagerungen gerichtet werden kann.

Der US-PS 4 799 754 läßt sich ferner entnehmen, daß für die angestrebte Beseitigung von Ablagerungen in Gefäßsystemen gepulstes Laserlicht erhöhter Energie besonders wirksam ist. Ein hierfür beispielsweise verwendbarer Laser ist der sogenannte Excimer-Laser, der als laserfähiges Medium ein Edelgashalogenid verwendet.

Es sind auch bereits Katheteranordnungen vorgeschlagen worden, bei denen die Laserlicht führenden Lichtleiter ringförmig um einen zentralen Innenkanal des Katheters angeordnet sind. Ein solcher Laserkatheter ist beispielsweise aus der DE-OS 37 39 965 bekannt. Die ringförmige Anordnung der Lichtleiter im distalen Ende des Katheters ermöglicht es, gerade den peripheren Bereich in einem Gefäß mit Laserenergie zu beaufschlagen, in dem sich die das Gefäß verengenden Ablagerungen im wesentlichen befinden.

Zum Vorschieben des Laserringkatheters an den zu rekanalisierenden Gefäßabschnitt wird erforderlichenfalls zuerst ein Führungskatheter in das Gefäßsystem eingeführt, durch dessen Innenraum dann erst der eigentliche Laserringkatheter bis zur Behandlungsstelle vorgeschoben wird. Der Ringkatheter trifft dann im allgemeinen auf die Gefäßablagerungen im zum behandelnden Abschnitt, wodurch sein weiteres Vorschieben in das zu rekanalisierende Gefäß durch dessen Verengung möglicherweise verhindert wird. Ein weiteres Vorschieben des Katheters ist erst in dem Maße möglich, in dem die Ablagerungen an den Gefäßwänden durch die Einwirkung der Laserstrahlung entfernt werden.

Der Innenkanal des Ringkatheters wird dabei nicht nur zum Durchleiten einer Spülflüssigkeit benutzt, er dient, wie in der DE-OS 37 39 965 beschrieben im allgemeinen auch dazu, einen Führungsdraht aufzunehmen, der bereits über das distale Ende des Kathethers hinaus in das verengte Gefäß eingeführt wird, um für den eigentlichen Laseringkatheter bei dessen weiterem Vorschub im Rahmen der Behandlung als zentrierende Führung zu dienen, unter anderem um zu vermeiden, daß durch Ablenken des distalen Katheterendes die Laserstrahlung ungewollt in schädigender Weise auf die organische Gefäßwand trifft.

Zur Durchführung der Behandlung mit Laserlicht ist es auch bereits bekannt, das Laserlicht gezielt nur in bestimmte Lichtleiter des Kranzes einzuleiten, wenn die Ablation der Gefäßablagerungen nur in einem bestimmten Umfangsbreich des Gefäßes erforderlich ist. Nicht für die Behandlung benötigte Lichtleiter können parallel mit nicht kohärentem Licht zur Beleuchtung der Behandlungsstelle beaufschlagt werden, die beispielsweise mit einem in den Innenkanal des Katheters eingeführten Lichtleiterendoskop beobachtet werden kann.

Schwierigkeiten in der Rekanalisationsbehandlung können beispielsweise dann auftreten, wenn das zu behandelnde Gefäß derart verengt ist, daß sich nicht einmal ein Führungsdraht für die weitere gezielte Positionierung des Katheters in das obstruierte Gefäß einführen läßt.

Der Erfindung liegt daher die Aufgabe zugrunde, ein Kathetersystem der eingangs bezeichneten Art derart weiterzubilden, daß mit ihm eine Rekanalisation auch von durch Ablagerungen praktisch vollständig verschlossene Gefäßabschnitte möglich ist.

Diese Aufgabe wird erfindungsgemäß dadurch gelöst, daß ein Pilotkatheter vorgesehen wird, der in den Innenkanal des Laserringkatheters einführbar und über dessen distales Ende vorschiebbar ist, und der seinerseits einen Lichtleiter enthält, der an seinem proximalen Ende mit Laserstrahlung beaufschlagbar ist.

Mit Hilfe dieses Pilotkatheters ist es möglich, erforderlichenfalls etappenweise einen zentralen Kanal durch die Ablagerungen im Gefäß "freizubohren", in den dann der Führungsdraht für das weitere Vorschieben des Hauptkatheters vorgetrieben werden kann. In bevorzugter Ausführungsform ist der Pilotkatheter selbst in einer Weise ausgebildet, daß er als Führungsdraht für den Hauptkatheter dienen kann. Er weist demzufolge einen ausreichend steifen Hohldraht auf, in dessen Lumen der Pilotlichtleiter angeordnet ist. Andererseits ist es natürlich auch möglich, zwischen einem Pilotkatheter und einem getrennt vorgesehenen Führungsdraht zu wechseln.

Der Pilotkatheter ist im Durchmesser zweckmäßigerweise derart bemessen, daß zur Innenwand des Innenkanals des Hauptkatheters ein ausreichender Abstand verbleibt, um den Innenkanal gleichzeitig auch als Leitung für ein Fluid, beispielsweise eine Spülflüssigkeit zu verwenden. Zu diesem Zweck ist der Innenkanal flüssigkeitsdicht ausgebildet und an seinem proximalen Ende mit einem Anschlußstutzen für eine entsprechende Fluidleitung versehen, der seitlich der Einführöffnung für den Pilotkatheter angeordnet ist. An der Einführöffnung für den Pilotkatheter sind dann entsprechende Abdichteinrichtungen zur Verhinderung eines Fluiddurchtritts, beispielsweise in Form einer Stopfbuchse vorgesehen.

Für den Fall, daß der Pilotkatheter selbst einen Hohldraht aufweist, kann dessen Lumen außer als zur Aufnahme des Pilotlichtleiters ebenfalls als Fluidkanal dienen, in welchem Fall am proximalen Ende des Pilotkatheters ebenfalls ein entsprechender Anschlußstutzen vorzusehen ist. Auf diese Weise läßt sich insgesamt eine Zirkulationsleitung für ein Spülfluid vorsehen.

Grundsätzlich können unterschiedliche Laserlichtquellen für die Beaufschlagung des Ringkatheters und des Pilotkatheters vorgesehen werden. Zweckmäßigerweise wird hierfür aber nur eine einzige Laserlichtquelle vorgesehen, in deren Strahlengang eine erste Strahlweicheneinrichtung, beispielsweise im Form eines für die Laserstrahlung teiltransparenten Ablenkspiegels vorgesehen ist, mit der ein für die Beaufschlagung des Pilotlichtleiters ausreichender Anteil an Laserenergie aus dem Hauptstrahl abzweigbar ist.

Um den Laserstrahl möglichst vollständig in die Eintrittsenden der Lichtleiter einkoppeln zu können, sind die Lichtleiter der Ringanordnung in bevorzugter Ausführungsform am proximalen Ende des Hauptkatheters seitlich aus dem Katheter herausgeführt und zu einer hexagonalen dichtesten Packung vereinigt, in der sie in der Eintrittsebene für die Laserstrahlung gefaßt sind. Zweckmäßigerweise ist in dem aus der Lasereinrichtung kommenden Laserstrahl eine dieser Packungskonfiguration entsprechende Begrenzungsblende vorgesehen, die optisch auf die Eintrittsebene der Lichtleiterpackung abgebildet wird.

In bevorzugter Ausführungsform sind die Lichtleiter am proximalen Ende des Hauptkatheters in gewisse Umfangsbereiche bildende Gruppen unterteilt, die sich selektiv mit Laserstrahlung beaufschlagen lassen. Diese Gruppen bilden zweckmäßigerweise zusammenhängende Flächenbereiche im Querschnitt der hexagonalen Packung am proximalen Ende des Katheters. Mittels im Strahlengang des Lasers vorgesehener Bereichsblenden lassen sich diese Flächenbereiche der Eintrittsebene der Lichtleiterpackung selektiv mit Laserlicht beaufschlagen.

In weiter bevorzugter Ausführungsform ist ferner zusätzlich eine Quelle für inkohärentes Beleuchtungs- oder Fluoreszenzanregungslicht vorgesehen, deren Strahlung wahlweise, zweckmäßigerweise ebenfalls über die erste Strahlweicheneinrichtung in die Lichtleiter des Hauptkatheters bzw. in bestimmte Lichtleitergruppen des Hauptkatheters und/oder in den Pilotlichtleiter einleitbar ist. Insofern das inkohärente Licht der Beleuchtung der Behandlungsstelle dient, kann diese durch ein Lichtleiterendoskop beobachtet werden, welches sich anstelle des Pilotkatheters in den Innenkanal des Hauptkatheters einführen läßt. Die Strahlenführung kann ferner derart ausgebildet sein, daß von der Behandlungstelle reflektiertes Licht einem Mehrkanalspektrometer zur Auswertung zuführbar ist.

Im folgenden wird anhand der beigefügten schematischen Zeichnungen eine bevorzugte Ausführungsform der erfindungsgemäßen Katheteranordnung im einzelnen noch näher erläutert.

In den Zeichnungen stellen dar:

Figur 1 eine schematische Gesamtansicht einer Katheteranordnung zum Rekanalisieren von Gefäßen des menschlichen Körpers,

Figur 2 die schematische Darstellung einer möglichen Strahlenführung zur Einkopplung von Laser- und anderem Licht in den Katheter wie zur Analyse von reflektiertem Licht,

Figur 3 einen Querschnitt durch den Katheter nach Figur 1 an dessen distalem Ende und

Figur 4 einen Querschnitt durch die hexagonale Packung der Lichtleiter des Ringkatheters am proximalen Einkopplungsende für das Laserlicht.

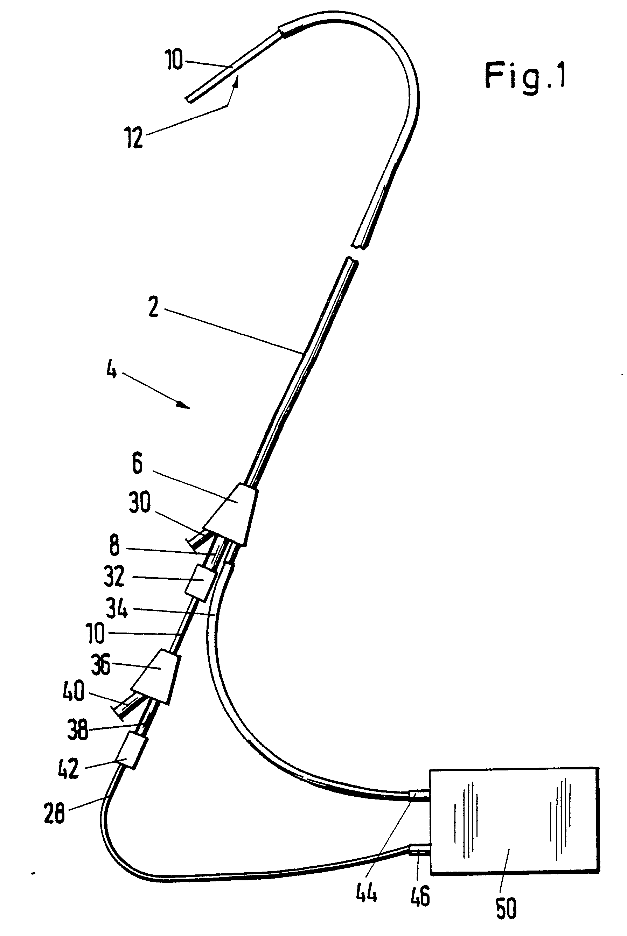

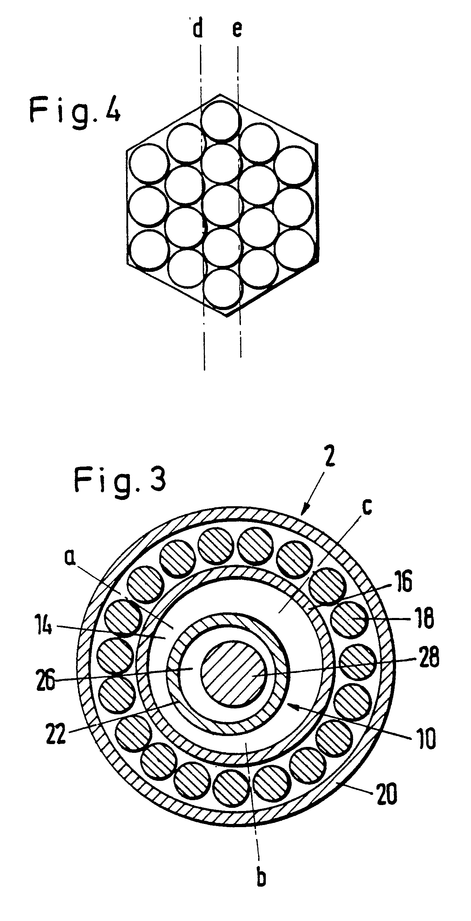

In Figur 1 ist die Katheteranordnung in Gesamtansicht dargestellt. Ein Hauptkatheter 2, der mit einem in Figur 1 nicht sichtbaren Innenkanal versehen ist, ist an seinem proximalen Ende mit einem Verzweigungsstück 6 versehen, welches unter anderem einen Einführstutzen 8 aufweist, der unmittelbar mit dem Innenkanal in Verbindung steht. Eingeführt in den Einführstutzen 8 ist ein Pilotkatheter 10, der am distalen Ende 12 der Katheteranordnung ein Stück aus dem Hauptkatheter 2 hinausragt. In Figur 3 ist ein Querschnitt durch die beiden Katheter in der Nähe des distalen Endes der Katheteranordnung dargestellt. Wie dort zu erkennen ist, besteht der Hauptkatheter 2 aus einem den Innenkanal 14 umschließenden Innenschlauch 16 um den herum kranzförmig Lichtleiter 18 angeordnet sind. Diese Gruppierung ist schließlich von einem Außenschlauch 20 umgeben.

Abgesehen von einer abweichenden Ausführung an den Enden des Hauptkatheters sind die Lichtleiter 18 nicht in ein bestimmtes Material eingebettet sondern befinden sich lose liegend zwischen dem Innenschlauch 16 und dem Außenschlauch 20 des Hauptkatheters. Aufgrund der dadurch gewährleisteten axialen Verschiebbarkeit zwischen den einzelnen Elementen erhält der Katheter eine bessere Flexibilität.

Im Innenkanal 14 des Hauptkatheters 2 befindet sich der Pilotkatheter 10. Er besteht seinerseits aus einem hohlen Führungsdraht 22, in dessen Lumen 26 ein einzelner Pilotlichtleiter 28 angeordnet ist. Es sei darauf hingewiesen, daß der Pilotkatheter 10 den Innenkanal 14 nicht vollständig ausfüllt, und daß auch der Pilotlichtleiter 28 das Lumen 26 des hohlen Führungsdrahtes 22 nicht voll ausfüllt. Hierdurch bleiben zwei Strömungskanäle erhalten, die beispielsweise für die Zirkulation von Spülflüssigkeit eingesetzt werden können. Hierfür ist es erforderlich, daß im wesentlichen der Innenschlauch 16 des Hauptkatheters, vorzugsweise aber auch der hohle Führungsdraht 22 des Pilotkatheters flüssigkeitsdicht ausgeführt sind. Dies kann erforderlichenfalls durch eine geeignete Beschichtung geschehen.

Es sei nun wieder die Figur 1 betrachtet. Außer dem Einführstutzen 8 für den Pilotkatheter weist das Verzweigungsstück 6 noch einen Anschlußstutzen 30 auf, der strömungsmäßig mit dem Innenkanal 14 des Hauptkatheters in Verbindung steht und dafür vorgesehen ist, mit einer (nicht dargestellten) Leitung beispielsweise für ein Spülfluid verbunden zu werden. Damit kein Fluid durch den Einführstutzen 8 austreten kann ist dieser an seiner Einführöffnung noch mit einer Abdichteinrichtung, beispielsweise in Form einer Stopfbuchse 32 versehen. Die Lichtleiter 18 sind innerhalb des Anschlußstückes 6 am proximalen Ende 4 des Katheters seitlich des Innenschlauches zusammengefaßt und als reines Lichtleiterbündel 34 aus dem Anschlußstück 6 herausgeführt.

Der Pilotkatheter 10 ist an seinem proximalen Ende ebenfalls mit einem Verzweigungsstück 36 versehen, welches einerseits einen Einführstutzen 38 für den Pilotlichtleiter 28 und andererseits einen Anschlußstutzen 40 aufweist, der mit dem Lumen 26 des Pilotkatheters in Strömungsverbindung steht und ebenfalls für den Anschluß einer (nicht dargestellten) Spülfluidleitung vorgesehen ist. Auch der Einführstutzen 38 ist mit einer fluidabdichtenden Einrichtung 42 versehen. Im Gegensatz zu der erforderlichen Verschiebbarkeit des Pilotkatheters im Hauptkatheter muß es nicht unbedingt erforderlich sein, den Pilotlichtleiter 28 im Pilotkatheter ebenfalls verschieben zu können. Er kann darin fest angeordnet sein, in welches Fall der Einführstutzen 38 eine feste Einführabdichtung darstellt.

Das Lichtleiterbündel 34 und der Pilotlichtleiter 28 sind an ihrem proximalen Enden mit Steckern 44 bzw. 46 versehen, mit denen sie an eine Zentraleinheit 50 angeschlossen sind, die eine Laserstrahlungsquelle und andere optische Hilfseinrichtungen enthält. Im Stecker 44 ist das Lichtleiterbündel 34 in haxagonal dichtester Packung gefaßt, wie dies in Figur 4 dargestellt ist.

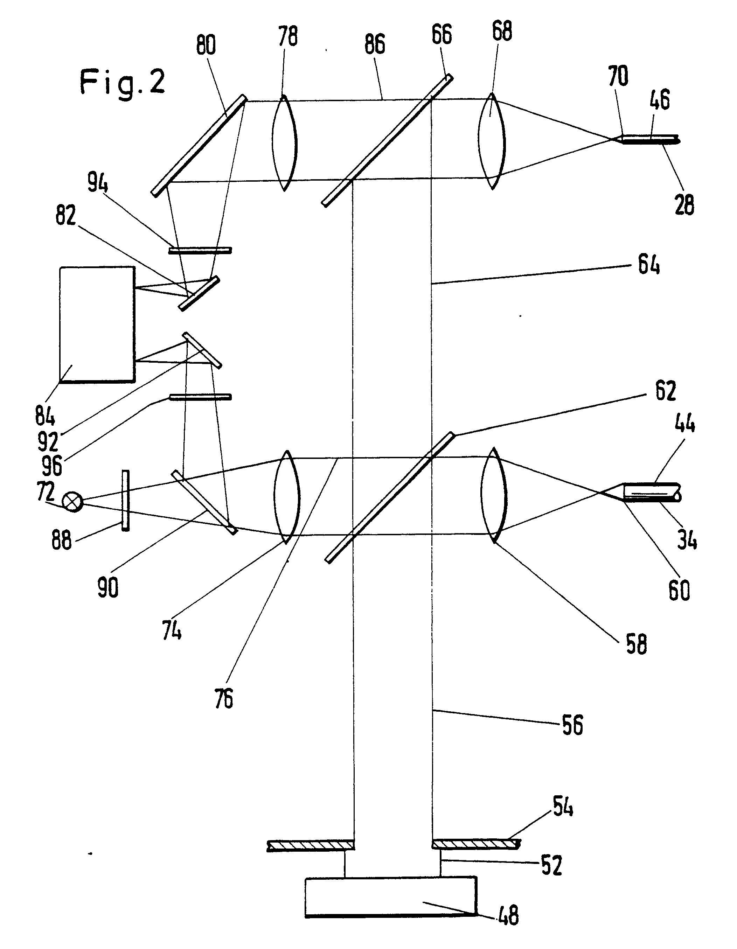

In Figur 2 ist ein Teil der optischen Anordnung aus der Zentraleinheit 50 schematisch dargestellt. Mit der Box 48 ist ein Laserstrahlungserzeuger angedeutet, der bekannt ist und in diesem Zusammenhang daher nicht im einzelnen beschrieben zu werden braucht. Für die hier beschriebene Anwendung wird bevorzugt gepulstes Laserlicht im Spektralbereich zwischen 240 und 3000 nm verwendet, wobei die Energiedichte im Einkopplungsquerschnitt der Laserstrahlung in die Lichtleiter des Katheters mindestens 20 J/cm$^2$ betragen sollte. Im Ausführungsbeispiel wurde ein Xenonchlorid Excimer-Laser mit einer Wellenlänge von 308 nm und einer zwischen 60 und 300 ns variablen Pulsbreite verwendet. Es sind beispielsweise aber auch Excimer-Laser einsetzbar, die andere Edelgashalogenide als laserfähiges Medium verwenden, wie auch Festkörperlaser, beispielsweise der Alexandrid-Laser oder der Holmium-Laser, da alle etwa die gleiche Schwellenergiedichte aufweisen.

Das aus dem Laserresonator 48 austretende Laserlichtbündel 52 wird entweder direkt oder nach Aufweitung durch ein Teleskop oder einen geeigneten Prismensatz, wobei anamorphotische Optik für asymmetrische Strahlprofile ausdrücklich mit einbezogen sein soll, durch eine an geeigneter Stelle positionierte körperliche Blende 54 geleitet, die Randstrahlen abschattet. Insbesondere dann, wenn das Laserlichtbündel vorher noch aufgeweitet wird, liegt die Energiedichte des Strahles in diesem Bereich noch auf einem so niedrigen Niveau, daß Manipulationen, wie beispielsweise durch die Blende 54 am Laserstrahl vorgenommen werden können, ohne daß Gefahr besteht, daß hier durch die Laserenergie die verwendeten Bauteile zerstört werden.

Der mittels der Blende 54 nunmehr geometrisch abgegrenzte Laserstrahl 56 wird mittels einer Konvexlinse 58 auf dem Eintrittsquerschnitt des im Stecker 44 gefaßten hexagonal gepackten Lichtleiterbündels 34 abgebildet. Die Blende 54 ist dabei so ausgebildet, daß sie in ihrer Außenkontur der Lichtleiterpackung geometrisch ähnlich ist, so daß ein unmittelbares Bild der Blende 54 durch die Optik auf den Eintrittsquerschnitt 60 des Lichtleiterbündels übertragen werden kann. Die Linse 58 ist derart angeordnet, daß sie ein reelles Bild der körperlichen Blende auf der Eintrittsfläche 60 des Lichtleiterbündels derart erzeugt, daß alle Fasern der hexagonal dichtesns Packung durch die Laserstrahlung beaufschlagt werden. Dabei ensteht eine Strahltaille des Laserbündels vor der Eintrittsfläche 60 des Lichtleiterbündels. Bei geeigneter Wahl der optisch geometrischen Daten kann dadurch eine Homogenisierung des transversalen Intensitätsprofils der Laserstrahlung erreicht werden. Bei einem typischen Ausführungsbeispiel beträgt der Durchmesser der Blende 54 etwa 10 mm und die Brennweite des abbildenden optischen Endgliedes 58 f = 100 mm. Bei einem Abstand von 11 f wird die Blende mit einem Durchmesser von etwa 1 mm auf der Eintrittsfläche 60 des Faserbündels abgebildet.

Auf dem Weg zur Optik 58 wird der Laserstrahl 56 durch einen Spiegel 62 umgelenkt. Dieser Spiegel 62 bildet eine erste Strahlweicheneinrichtung, indem er derart ausgebildet ist, daß er in Abhängigkeit von der verfügbaren Laserleistung zwischen 50 und 80 % der Laserstrahlung auf die abbildende Optik 58 reflektiert, gleichzeitig aber zwischen 20 und 50 % des primären Laserstrahls 56 hindurch läßt, wodurch ein Sekundärstrahl 64 entsteht. Dieser Sekundärstrahl 64 trifft auf einen weiteren Umlenkspiegel 66, durch den der Strahl auf eine Optik oder Linse 68 geleitet wird, die in entsprechender Weise wie die Linse 58 dazu dient, den Sekundärstrahl 64 auf die Eintrittsfläche 70 des im Stecker 46 gefaßten Pilotlichtleiters 28 zu konzentrieren. Auf diese Weise wird eine einziger Laserlichtquelle dazu genutzt, Laserstrahlung sowohl in das Lichtleiterbündel 34 des Haupt- oder Ringkatheters wie auch in den Pilotlichtleiter 28 des Pilotkatheters 10 einzukoppeln. Die Brennweite der Linse 68 ist kürzer als diejenige der Linse 58 und beträgt im Ausführungsbeispiel 50 mm.

In der optischen Achse des Lichtleiterbündels 34 ist ferner eine Lichtquelle 72 für inkohärentes Licht angeordnet, welches beispielsweise für Beleuchtungszwecke zur Verfügung steht. Von der Lichtquelle 72 ausgesandte Strahlung wird durch eine aus einer Konweklinse 74 bestehende Optik zuerst Parallel ausgerichtet und der Querschnitt des parallel gerichteten Lichtstrahles 76 kann dann mittels der Optik 58 in verkleinerter Form auf dem Eintrittsquerschnitt 60 des Lichtleiterbündels 34 abgebildet werden. Auf diese Weise können die Lichtleiter des Hauptkatheters mit Licht zur Beleuchtung der Behandlungsstelle beaufschlagt werden.

Die erste Strahlweicheneinrichtung in Form des Spiegels 62 ist derart ausgebildet, daß der Spiegel für Strahlung im Spektralbereich <400 nm, das heißt also für die verwendete Laserstrahlung im oben angegebenen Teilungsverhältnis teiltransparent ist, jedoch für Strahlung im Spektralbereich zwischen 400 und 700 nm vollständig durchlässig ist, so daß die Strahlung der Lichtquelle 72 ungehindert durch den Spiegel 62 hindurchtreten kann. Insofern es erwünscht sein sollte, ein Teil der inkohärenten Beleuchtungsstrahlung auch auf den Pilotlichtleiter umleiten zu können, könnte der Spiegel 62 auch für das Beleuchtungslicht teilreflektierend ausgebildet werden, so daß ein Teil der inkohärenten Strahlung über den Spiegel 66 in den Pilotlichtleiter 28 eingekoppelt werden kann.

Im Normalfall wird aber derart gearbeitet, das Beleuchtungslicht durch den Spiegel 62 hindurch in das Lichtleiterbündel 32 des Hauptkatheters 2 eingeleitet wird. Zur Beobachtung der Behandlungsstelle kann dann anstelle des Pilotkatheters 10 ein Endoskop in den Innenkanal 14 der Katheteranordnung eingeführt werden. Das System ist aber auch derart ausgebildet, daß die während der Laserstrahlungsbehandlung am Behandlungsort erzeugte Sekundärstrahlung einer Analyse unterzogen werden kann, um daraus Rückschlüsse auf die Vorgänge am Behandlungsort ziehen zu können. Zur Rückleitung der Sekundärstrahlung kann entweder das in den Innenkanal 14 eingeführte Endoskop, aber unter bestimmten Bedingungen auch der Pilotlichtleiter verwendet werden, wenn sich der Pilotkatheter im Innenkanal 14 befindet. Zur Erfassung der Sekundärstrahlung ist der Umlenkspiegel 66 für diese Sekundärstrahlung transparent ausgebildet, so daß die Sekundärstrahlung 86 mittels einer Linse 78 und nach Umlenkung durch zwei Spiegel 80 und 82 auf den Eingang eines Simultanspektrometers 84 abgebildet werden kann.

Die Anordnung bietet aber nicht nur die Möglichkeit, durch das behandelnde Laserlicht erzeugte Sekundärstrahlung analysieren zu können, es ist mit dem System auch möglich, eine ganz bestimmte Fluoreszenzanregungsstrahlung über das Lichtleiterbündel 34 an den Behandlungsort zu leiten. Hierfür ist im Strahlengang der Lichtquelle 72 eine Wechselfiltereinrichtung 88 vorgesehen, die beispielsweise mit einem Fluoreszenzanregungsfilter bestückt sein kann, so daß lediglich Licht im Spektralbereich zwischen 300 und 440 nm zur Fluoreszenzanregung simultan mit der Laserstrahlung durch das Lichtleiterbündel 34 übertragen werden kann. Das Fluoreszenzsignal kann dann über den Sekundärstrahlungsweg durch den Pilotkatheter oder das Endoskop, welches in diesem Fall selbstverständlich ohne Okular zu verwenden ist, im Spektrometer 84 analysiert werden. Mit einem halbdurchlässigen Spiegel 90 im Strahlengang der Lichtquelle 72 ist es weiterhin möglich, einen Anteil der Fluoreszenzanregungsstrahlung über einen Umlenkspiegel 92 einer weiteren Eingangsblende des Spektrometers als Vergleichsstrahlung zuzuführen. Vor den Eintrittsblenden des Spektrometers 84 sind noch Filteranordnungen 94 und 94 vorgesehen, die beispielsweise mit UV Filtern bestückt sein können.

Zur Analyse der Behandlungsvorgänge können im Simultanspektrometer beispielsweise die Plas-maemissionskennlinien des bei dem Behandlungs-prozess enstehenden Plasmas analysiert werden, bzw. kann die aufgrund des Raman-Effektes verschobene Streustrahlung, die gewebespezifische Informationen beinhalten, ausgewertet werden. Grundsätzlich ist auch die im Gewebe entstehende Fluoreszenzstrahlung für eine Analyse geeignet. Die Sekundärstrahlung kann erforderlichenfalls auch über das Lichtleiterbündels des Hauptkathethers 2 bzw. über Teile davon zurückgeleitet werden.

In bevorzugter Ausführungsform sind die Lichtleiter 28 in ihrer hexagonalen Packung im Stecker 44 derart angeordnet, daß bestimmte, einen zusammenhängenden Querschnittsflächenbereich dieser Packung bildende Lichtleitergruppen mit Lichtleitergruppen am Umfang des Hauptkatheters 2 an dessen distalem Ende 12 übereinstimmen. Dieser Zusammenmhang ist in den Figuren 3 und 4 verdeutlicht. So bilden beispielsweise die 7 Lichtleiter zwischen den Linien a und b in Figur 3 die Gruppe von Lichtleitern links der Linie d in der Figur 4. Entsprechend bilden die 5 Lichtleiter zwischen den Linien a und c· in Figur 3 die Gruppe von Lichtleitern zwischen den Linien d und e in Figur 4. Durch entsprechende in der Blendeneinrichtung 54 angeordnete Steckblenden, mit denen sich Bereiche der Blendenfläche ausblenden lassen, welche den Lichtleitergruppen gemäß Figur 4 entsprechen, lassen sich diese Gruppe selektiv mit Laserstrahlung beaufschlagen. So ist es beispielsweise möglich, nur eine einzige Umfangsgruppe von Lichtleitern mit Laserlicht zur Behandlung zu beaufschlagen, wenn nur an einem bestimmten Umfangsbereich des Gefäßes Ablagerungen zu entfernen sind, während die restlichen Lichtleiter noch zur Übertragung von Beleuchtungsstrahlen verwendet werden können. Die Aufteilung der Lichtleiter in bestimmte Gruppen gemäß den Figuren 3 und 4 ist lediglich als beispielhaft anzusehen. Sie kann auch in jeder beliebigen anderen Anordnung vorgenommen werden.

Am distalen Ende können die Lichtleiter des Hauptkatheters 2 in ein geeignetes optisches Abschlußstück münden. Die Lichtleiter können auch gruppenweise in einer Aufteilung, wie sie der selektiven Beaufschlagbarkeit mit Laserstrahlung gemäß den Figuren 3 und 4 entspricht, durch einen elastischen Werkstoff miteinander verbunden sein, der es ermöglicht, den Hauptkatheter am distalen Ende aufzuweiten, so daß die Laserstrahlung mehr in den peripheren Bereich des zu behandelnden Gefäßes geleitet werden kann. Die Art und Weise, wie die Lichtleiter zweckmäßigerweise am distalen wie auch am proximalen Ende der Katheteranordnung gefaßt sind, ist jedoch nicht Gegenstand dieser Anmeldung.

Der große Vorteil der beschriebenen Anordnung liegt darin, daß trotz der vielseitigen Funktionen der Katheteranordnung der Durchmesser des Hauptkatheters in einem Bereich zwischen French 4 und French 8 verhältnismäßig gering gehalten werden kann. Dies wird im wesentlichen dadurch erreicht, daß einerseits die Lichtleiter im Ringkatheter 2 selektiv beaufschlagbar sind und mehrere Funktionen ausführen können, im wesentlichen aber dadurch, daß der Innenkanal dazu benutzt wird, jeweils nur das jenige Zusatzelement in den Hauptkatheter einzuführen, welches gerade zur Behandlung benötigt wird, ohne daß der Katheter konstruktiv durch das Vorsehen unterschiedlichster technischer Mittel in einer einzigen festen Anordnung belastet wird. So macht es die Beschränkung des Kathetersystems auf die vorstehend genannten Durchmesser überhaupt erst möglich, die angestrebten Behandlungsmaßnahmen auch in kleineren Gefäßen durchzuführen, wie sie im Bereich der Unterschenkeletage und der Koronarien des Her-zens auftreten.

## Ansprüche

1. Kathetersystem, insbesondere für die Gefäßrekanalisation im menschlichen Körper, mit einem flexiblen Hauptkatheter, der einen durchgehenden Innenkanal aufweist, in den gegebenenfalls ein Führungsdraht einführbar ist, und der einen den Innenkanal umgebenden Kranz von Lichtleitern enthält, die am proximalen Ende des Katheters zumindest gruppenweise getrennt mit Laserstrahlung beaufschlagbar sind, und mit mindestens einer Quelle für gepulste Laserstrahlung sowie einer Einkoppelanordnung zum Einkoppeln der Laserstrahlung in die Lichtleitfasern,
dadurch gekennzeichnet,
daß ein in den Innenkanal (14) des Hauptkatheters (2) einführbarer und über dessen distales Ende (12) hinaus vorschiebbarer Pilotkatheter (10) vorgesehen ist, der einen Pilotlichtleiter (28) enthält, in dessen Proximales Ende pepulstes Laserlicht einkoppelbar ist.

2. Kathetersystem nach Anspruch 1, dadurch gekennzeichnet, daß eine einzige Quelle (48) für Laserlicht vorgesehen ist und die Einkoppelanordnung eine erste Strahlweicheneinrichtung (62) im Strahlenweg der Laserstrahlung (56) aufweist, mit der ein Anteil der Laserstrahlung für die Einkoppelung in den Pilotkatheter (10) abzweigbar ist.

3. Kathetersystem nach Anspruch 2, dadurch gekennzeichnet, daß mit der ersten Strahlweicheneinrichtung (62) die Laserstrahlung (56) im Verhältnis der Querschnitte (60,70) der mit Laserstrahlung zu beaufschlagenden Lichtleiter (34,28) des Hauptkatheters (2) und des Pilotkatheters (10) aufteilbar ist.

4. Kathetersystem nach Anspruch 2 oder 3, dadurch gekennzeichnet, daß eine Quelle (72) für inkohärentes Beleuchtungs- oder Fluoreszenzanregungslicht vorgesehen ist, welches über die erste Strahlweicheneinrichtung (62) in den Strahlengang zu den Lichtleitern (18) des Hauptkatheters (2) einkoppelbar ist.

5. Kathetersystem nach mindestens einem der Ansprüche 2-4, dadurch gekennzeichnet, daß die Lichtleiter (28) des Hauptkatheters (2) an ihrem Proximalen Eintrittsende in hexagonal dichtester Packung angeordnet sind, und daß zwischen der Quelle (48) für das gepulste Laserlicht und der ersten Strahlweicheneinrichtung (62) eine den Laserstrahl (52) begrenzende Blende (54) angeordnet ist, deren Öffnung mittels optischer Ankoppelungselemente (58,68) jeweils verkleinert auf die Eintrittsquerschnitte (60,70) der hexagonalen Packung der Lichtleiter (18) des Hauptkatheters (2) bzw. des Lichtleiters (28) des Pilotkatheters (10) abgebildet wird.

6. Kathetersystem nach Anspruch 5, dadurch gekennzeichnet, daß die Öffnung der Blende (54) in Teilbereichen ihres Querschnitts durch Blendenelemente verschließbar ist, wobei den Querschnittsteilbereichen bestimmte Lichtleitergruppen des Lichtleiterkranzes des Hauptkatheters zugeordnet sind.

7. Kathetersystem nach mindestens einem der Ansprüche 1-6, dadurch gekennzeichnet, daß die erste Strahlweicheneinrichtung (62) so ausgebildet ist, daß die durch die Lichtleiter (18) des Hauptkatheters (2) reflektierte Strahlung von der in den Hauptkatheter eintretenden Strahlung aufgrund unterschiedlicher Wellenlänge trennbar ist, und daß Mittel (74,90,92) vorgesehen sind, die reflektierte Strahlung einer Auswerteinheit (84), beispielsweise einem Mehrkanalspektrometer zuzuführen.

8. Kathetersystem nach Anspruch 7, dadurch gekennzeichnet, daß eine zweite Strahlweicheneinrichtung (66) vorgesehen ist, mit der in den Pilotlichtleiter (28) des Pilotkatheters (10) reflektierte Strahlung (86) einer Auswerteinheit (84) zuführbar ist.

9. Kathetersystem nach mindestens einem der Ansprüche 1 8, dadurch gekennzeichnet, daß der Innenkanal (14) des Hauptkatheters (2) fluiddicht ausgeführt und an seinem proximalen Ende mit einem Anschlußstutzen (3) für die Verbindung mit einer Spülfluidleitung versehen ist, und daß die Einführöffnung (8) am Proximalen Ende zum Einführen des Pilotkatheters (10) in den Innenkanal (14) mit einer Einrichtung (32) zum Abdichten gegen Fluiddurchtritt versehen ist.

10. Kathetersystem nach mindestens einem der Ansprüche 1-9, dadurch gekennzeichnet, daß der Pilotkatheter (10) in einer Weise bemessen ist, daß zwischen seiner Außenwand und der Innenwand des Innenkanals (14) ein Zwischenraum verbleibt, der als Kanal für das Durchleiten eines Spülfluids ausreicht.

11. Kathetersystem nach mindestens einem der Ansprüche 1-10, dadurch gekennzeichnet, daß der Pilotkatheter (10) seinerseits einen hohlen Führungsdraht (22) aufweist, in dem der Pilotlichtleiter (28) mit Spiel angeordnet ist, und daß der einen zweiten Fluidkanal bildende Abstandsraum zwischen Führungsdraht (22) und Pilotlichtleiter (28) am proximalen Ende des Pilotkatheters (10) mit einem Anschlußstutzen (40) für eine Spülfluidlösung versehen ist.

12. Kathetersystem nach mindestens einem der Ansprüche 1-11, dadurch gekennzeichnet, daß eine Bildübertragungseinrichtung vorgesehen ist, die anstelle des Pilotkatheters (10) in den Hauptkatheter (2) einführbar ist.

Fig.1

# Fig.2

Fig.4

Fig.3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | EP-A-0 297 190 (MCM) * Spalte 6, Zeilen 17-40; Spalte 8, Zeilen 8-22; Spalte 9, Zeilen 15-29; Spalte 10, Zeile 50 - Spalte 11, Zeile 3; Figuren 5,11-16 * | 1,9,10,12 | A 61 B 17/22 |
| Y | --- | 2-8,11 | |
| X,P | EP-A-0 355 996 (ADVANCED INT. SYSTEMS) * Anspruch 12; Figur 11 * --- | 1 | |
| Y,D | EP-A-0 195 375 (M.I.T.) * Seite 43, Zeilen 3-18; Seite 44, Zeilen 14-17; Seite 48, Zeile 6 - Seite 49, Zeile 7; Figuren 11B,19,21 * --- | 2-8 | |
| Y | US-A-4 768 858 (HUSSEIN) * Spalte 4, Zeilen 43-55; Figur 2 * --- | 11 | |
| A | US-A-4 296 319 (FRANKS et al.) * Spalte 3, Zeile 67 - Spalte 4, Zeile 23; Figur 3 * ----- | 5,6 | |
| | | | **RECHERCHIERTE SACHGEBIETE (Int. Cl.5)** |
| | | | A 61 B |

Der vorliegende Recherchenbericht wurde für alle Patentansprüche erstellt

| Recherchenort | Abschlußdatum der Recherche | Prüfer |
|---|---|---|
| DEN HAAG | 13-06-1990 | MOERS R.J. |

EPO FORM 1503 03.82 (P0403)